# EUROPEAN PATENT APPLICATION

(11) **EP 2 805 936 A1**
(43) Date of publication of application: **26.11.2014**
(21) Application number: 13168413.6
(22) Date of filing: 20.05.2013
(51) Int. Cl.: C07C 231/12, C07C 235/40, C07C 237/24

(54) **Process for preparing levomilnacipran HCL**

(71) Applicant: Cosma S.p.A., 24040 Ciserano (IT)
(72) Inventor: Celestini, Paolo, 24058 Romano di Lombardia (IT); Baretti, Sergio, 24040 Boltiere (IT); Pizzatti, Enrica, 23020 Poggiridenti (IT)
(74) Representative: Cattaneo, Elisabetta

(57) **Abstract**

The invention relates to one-pot process for preparing (1S,2R)-1-phenyl-1-(N,N-diethylaminocarbonyl)-2-aminomethylcyclopropane of formula (I) comprising the step of reacting (1S,2R)-N,N-diethyl-2-(hydroxymethyl)-1-phenylcyclopropanecarboxamide successively with the following reactants 1) triethyl orthoformate and methanesulfonic acid or triethylamine and methanesulfonyl chloride, 2) a phthalimidating agent, 3) aqueous EtNH₂, wherein the reaction is carried out in toluene. In another aspect the invention concerns a process for preparing (1S,2R)-N,N-diethyl-2-(hydroxymethyl)-1-phenylcyclopropanecarboxamide trough a step of crystallization of (1S,5R)-1-phenyl-3-oxabicyclo[3.1.0]hexan-2-one.

## Description

### FIELD OF INVENTION

The invention concerns a process for producing (1S,2R)-milnacipran, i.e. (1S,2R)-1-phenyl-1-(N,N-diethylaminocarbonyl)-2-aminomethylcyclopropane of formula (I) 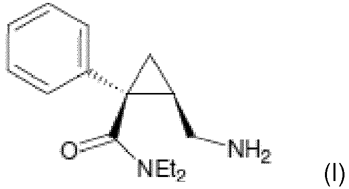 or a pharmaceutically acceptable addition salt, preferably the hydrochloride salt.

### STATE OF THE ART

Milnacipran is an antidepressant inhibiting recapture of serotonin-noradrenalin recommended in the treatment of the depression.

Processes for producing milnacipran racemate are described in the art. In EP1845084, milnacipran and milnacipran hydrochloride are obtained without using thionyl chloride. Specifically the process provides for reacting 1-phenyl-1-(N,N-diethylaminocarbonyl)-2-hydroxymethylcyclopropane, also known as milna-alcohol, with an orthoester and a Brönsted acid, thus obtaining an iminium salt of Formula 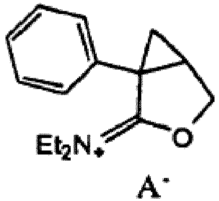 wherein A⁻ is the conjugated base of a Brönsted acid, preferably CH₃SO₃ The iminium salt is then converted to Milna-phthalimido (1-phenyl-1-(N,N-diethylaminocarbonyl)-2-phthalimidomethylcyclopropane) through a phthalimidating agent and after reaction with aqueous methylamine milnacipran is obtained.

It is also known that the enantiomer (1S,2R)-milnacipran is more active than the racemic mixture. Resolutions of the two enantiomers or enantio-selective syntheses have been described, even if they resulted to be expensive and not advantageous from an industrial point of view.

In WO2010/086394 a method for the synthesis of (1S,2R) enantiomeric form as hydrochloride salt is described. The process comprises six steps: a) reaction of phenylacetonitrile and of (R)-(-)epichlorhydrin in the presence of a base containing an alkaline metal, followed by a basic treatment, and then by an acid treatment in order to obtain milna-lactone; b) reaction of the milna-lactone with MNEt₂, wherein M is an alkaline metal, or NHEt₂ in the presence of a Lewis acid-amine complex, preferably diethylamine, in order to obtain an amide-alcohol; c) reaction with thionyl chloride in order to get the chlorinated amide; d) reaction of the chlorinated amide with a phthalimide salt to obtain the phthalimido derivative which is hydrolyzed in step e) to have (1S, 2R)-milnacipran. In step (f) (1S,2R)-milnacipran is converted in salt in the presence of a pharmaceutically acceptable acid.

Even if this process allows the active enantiomer to be obtained, it uses thionyl chloride which generates a lot of impurities during the process, thus requiring extraction steps and or volume concentrations.

The inventors tried to repeat the process as per claim 1 of EP1845084 using dimethylformamide for achieving milna-phthalimide and realized that it was necessary to isolate and purify milna-phthalimide in order to proceed with the subsequent step.

The object of the present invention is therefore to provide (1S,2R)-milnacipran or its pharmaceutical salt in a convenient and simple way from a industrial point of view.

### SUMMARY OF INVENTION

The above object has been achieved by providing a one-pot process for preparing (1S,2R)-milnacipran, i.e. (1S,2R)-1-phenyl-1-(N,N-diethylaminocarbonyl)-2-aminomethylcyclopropane comprising the step of reacting (1S,2R)-N,N-diethyl-2-(hydroxymethyl)-1-phenylcyclopropanecarboxamide successively with the following reactants 1) triethyl orthoformate and methanesulfonic acid or triethylamine and methanesulfonyl chloride, 2) a phthalimidating agent, 3) aqueous EtNH₂,
wherein the reaction is carried out in toluene.

In another aspect the invention concerns the preparation of milnacipran hydrochloride enantiomer from the (1S,2R)-milnacipran through process for preparing (1S,2R)-1-phenyl-1-(N,N-diethylaminocarbonyl)-2-aminomethylcyclopropane hydrochloride with a combination of a first solvent and a second solvent comprising reacting (1S,2R)-1-phenyl-1-(N,N-diethylaminocarbonyl)-2-aminomethylcyclopropane with hydrochloride acid in the presence of the first solvent and the mixture so obtained is treated with the second solvent, being the combination selected from the group consisting of:
a) first solvent: methyl isobutyl ketone (MIBK) and second solvent: acetone,
b) first solvent: isopropanol and second solvent: methyl isobutyl ketone (MIBK)
c) first solvent: isobutanol and second solvent ethyl acetate, and
d) first solvent: isobutanol and second solvent isobutyl acetate.

In a still another aspect the invention concerns the preparation of levomilna-alcohol, i.e. (1S,2R)-N,N-diethyl-2-(hydroxymethyl)-1-phenylcyclopropanecarboxamide of Formula (II) 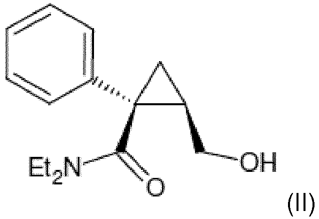 through a process comprising the following step:
- reacting phenylacetonitrile with R(-)epichlorhydrin in the presence of a base containing an alkaline metal, followed by treatment of an alkaline metal hydroxide and then by a treatment with an acid, thus obtaining levomilna-lactone, i.e. (1S,5R)-1-phenyl-3-oxabicyclo[3.1.0]hexan-2-one of formula (III); 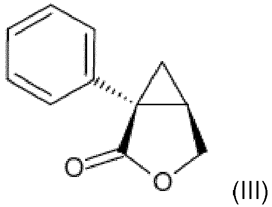
- crystallizing the levomilna-lactone as crude oil in a mixture of isopropyl alcohol and water;
- reaction of the crystallized milna-lactone with diethylamine in the presence of Lewis acid-amine complex in dichloromethane. Advantageously the process for obtaining levo-milnacipran base, with the further salification step to levomilnacipran hydrochloride allows to obtain levomilnacipran enantiomer or its pharmaceutically acceptable salt in a convenient way from an industrial point of view avoiding the use of thionyl chloride, since it does not contemplate a lot of separation, concentration and purification steps, even if it achieves a final product with an high chiral purity.

More advantageously if the process of the invention for producing levo-milnacipran base starts from levo-milna alcohol obtained according to the process comprising the step of crystallizing the levomilna lactone of Formula (III) the final product has a very high purity due to the very high chiral purity of the starting levo-milna alcohol.

### DESCRIPTION OF THE DRAWINGS

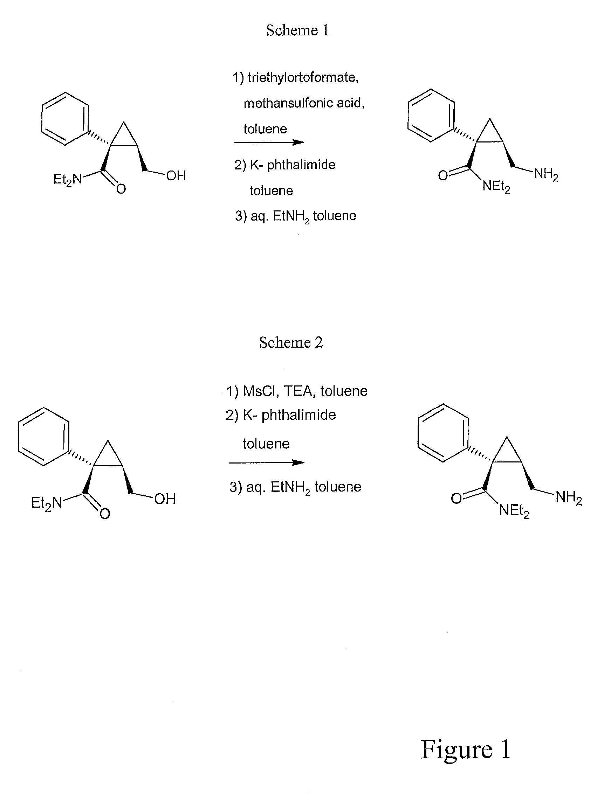

Figure 1 reports the detailed scheme 1 and 2 of the two preferred embodiment of the invention;

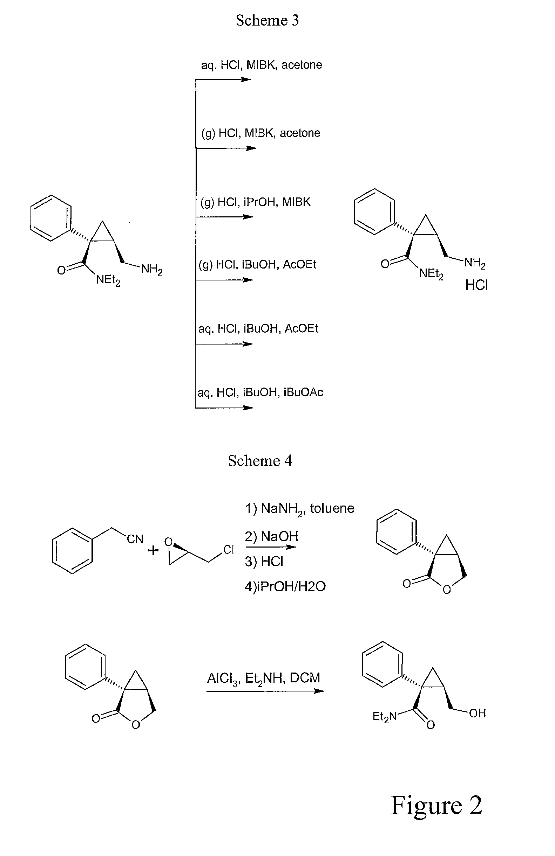

Figure 2 reports the detailed scheme 3 of the process for producing (1S,2R)-milnacipran hydrochloride and the detailed scheme 4 of the process for producing (1S,2R)-milna-alcohol.

### DETAILED DESCRIPTION OF THE INVENTION

The invention concerns a one-pot process for preparing (1S,2R)-milnacipran comprising the step of reacting (1S,2R)-N,N-diethyl-2-(hydroxymethyl)-1-phenylcyclopropanecarboxamide successively with the following reactants 1) triethyl orthoformate and methanesulfonic acid or triethylamine and methanesulfonyl chloride, 2) a phthalimidating agent, 3) aqueous EtNH₂, wherein the reaction is carried out in toluene.

According to the invention (1S,2R)-N,N-diethyl-2-(hydroxymethyl)-1-phenylcyclopropanecarboxamide i.e. levomilna alcohol of formula (II) is successively reacted with the following reactants 1) triethyl orthoformate and methanesulfonic acid or triethylamine and methanesulfonyl chloride, 2) a phthalimidating agent, 3) aqueous EtNH₂.

In a first embodiment of the invention the reactant 1) is triethyl orthoformate and methanesulfonic acid and in a second embodiment of the invention the reactant 1) is triethylamine and methanesulfonyl chloride, which are both used in toluene. With the first addition of the reactants in toluene in both the embodiments of the invention the separation and purification of the levo-milna iminium methanesulfonate compound of formula

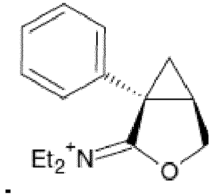

. OMs⁻

,
either in the form of a compound or as an intermediate, are advantageously avoided.

The phthalimidating agent 2) is preferably potassium phthalimide. Another suitable phthalimidating agent is sodium phthalimide

The reactant 3) is aqueous ethylamine.

Toluene can be used as the single solvent of the process to obtain one-pot, i.e. without separating the product obtained after each addition of 1) to 3).

When the inventors studied both one-pot embodiments they tried to use as the sole and single solvent dimethylformamide instead of toluene, but they found out that milna-phthalimido had to be precipitated and separated in order to proceed for achieving milnacipran base. They also tried to use ketones and esters, but the final yields were not satisfactory.

The final product (1S,2R)-milnacipran base can be further salified, thus obtaining a pharmaceutically acceptable salt in the presence of a pharmaceutically acceptable acid. This can be an inorganic acid such as hydrochloric acid, hydrobromic acid, sulphuric acid, nitric acid, phosphoric acid or organic acids such as acetic acid, benzene-sulfonic acid, benzoic acid, camphor-sulfonic acid, citric acid, ethane-sulfonic acid, fumaric acid, glucoheptonic acid, gluconic acid, glutamic acid, glycolic acid, hydroxynaphthoic acid, 2-hydroxyethane-sulphonic acid, lactic acid, maleic acid, malic acid, mandelic acid, methane-sulphonic acid, muconic acid, 2-naphthalene-sulfonic acid, propionic acid, salicylic acid, succinic acid, dibenzoyl-L-tartaric acid, tartaric acid, p-toluene-sulphonic acid, trimethylacetic acid, trifluoroacetic acid.

The pharmaceutically acceptable acid is preferably an inorganic acid, more preferably hydrochloric acid. For example (1S,2R)-milnacipran hydrochloride can be obtained by reacting hydrochloric acid in the presence of isopropylalcohol and ethylacetate.

In another aspect the invention therefore concerns process for preparing (1S,2R)-1-phenyl-1-(N,N-diethylaminocarbonyl)-2-aminomethylcyclopropane hydrochloride with a combination of a first solvent and a second solvent comprising reacting (1S,2R)-1-phenyl-1-(N,N-diethylaminocarbonyl)-2-aminomethylcyclopropane with hydrochloride acid in the presence of the first solvent and the mixture so obtained is treated with the second solvent, being the combination selected from the group consisting of:
a) first solvent: methyl isobutyl ketone (MIBK) and second solvent: acetone,
b) first solvent: isopropanol and second solvent: methyl isobutyl ketone (MIBK)
c) first solvent: isobutanol and second solvent ethyl acetate, and
d) first solvent: isobutanol and second solvent isobutyl acetate.

The process of salification according to the invention allows to obtain an hydrochloride salt of (1S,2R)-milnacipran very pure in a very convenient way from an industrial point of view as it will be clear from the following experimental part. Furthermore the process of for preparing (1S,2R)-milnacipran hydrochloride of the invention allows advantageously to add hydrochloric acid as either aqueous HCl or gaseous HCl.

The invention concerns also the preparation of levomilna-alcohol, i.e. (1S,2R)-N,N-diethyl-2-(hydroxymethyl)-1-phenylcyclopropanecarboxamide of Formula (II) 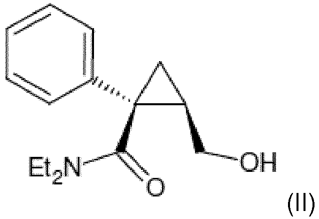 through a process comprising the following step:
i) reacting phenylacetonitrile with R(-) epichlorhydrin in the presence of a base containing an alkaline metal, followed by treatment of an alkaline metal hydroxide and then by a treatment with an acid, thus obtaining levomilna-lactone, i.e. (1S,5R)-1-phenyl-3-oxabicyclo[3.1.0]hexan-2-one of formula (III); 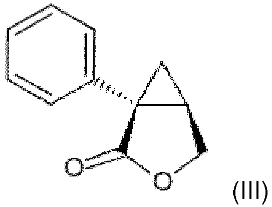
ii) crystallizing the levomilna-lactone as crude oil in a mixture of isopropyl alcohol and water;
iii) reaction of the crystallized milna-lactone with diethylamine in the presence of Lewis acid-amine complex in dichloromethane

Preferably, in step i) a base containing an alkaline metal can be NaH, NaNH₂, potassium or litium hexamethyldisilazane (KHMDS or LiHMDS), butyllithium,hexyl lithium, sodium or potassium tertiobutylate or lithium diisopropylamide (LDA). Preferably the base containing alkaline metal is NaNH, which is more preferably reacted in the presence of toluene.

The alkaline metal hydroxide is preferably NaOH or KOH, more preferably both as aqueous solutions, still more preferably it is NaOH or an aqueous solution of NaOH. As subsequent acid treatment hydrochloric acid can be used, preferably as an aqueous solution. More preferably an aqueous solution of 30% of NaOH and an aqueous solution of 36-37% of HCl are used.

In step ii) the compound of formula (III) is crystallized with isopropyl alcohol and water. This specific crystallizing mixture is extremely advantageous since it allows to increase the chiral purity from about 98.2% to 99.8%, thus allowing a levomilna alcohol of step (iii), which is then used for the process of the invention, to be obtained in a purer way, thus ameliorating the productivity and the quality of the products involved in the present invention.

In step iii) levo-milna alcohol, i.e. (1S,2R)-N,N-diethyl-2-(hydroxymethyl)-1-phenylcyclopropanecarboxamide of Formula (II) is obtained by the treatment of levo-milna lactone with diethylamine in the presence of Lewis acid-amine complex. Preferably Lewis acid-amine complex is AlCl₃-NHEt₂.

The invention will be better understood in the light of the non-limitative experimental part.

### Example 1

### (Process of the invention for synthesizing levomilnacipran base via 1) triethyl orthoformate and methanesulfonic acid)

(1S,2R)-N,N-diethyl-2-(hydroxymethyl)-1-phenylcyclopropanecarboxamide (49.4 g) was charged into a 4 neck round bottom flask, under nitrogen atmosphere, at 20-25 °C, followed by toluene (24.7 ml) and triethyl orthoformate (44.4 g). Methanesulfonic acid (25.24 g) was added dropwise, maintaining the temperature at 25±5 °C. The mixture was stirred at 25±5 °C for 15 hours, then it was charged over a period of about 1 hour on a slurry of potassium phthalimide (48.16 g) in toluene (366 ml) at 55-60 °C. The reaction was stirred at that temperature for at least 0.5 hour.

The mixture was cooled to 15-20 °C and aq. 70% EtNH₂ (89.96 g) was added, maintaining the same temperature. The reaction mixture was hold at 15-20 °C for 1 h, then heated to 40-42 °C for 1 hour, then water (224 ml) was added and then the mixture was hold at 40-42 °C for 12 hours. 30% NaOH (25.32 g) was added and the organic layer was separated. The aqueous layer was further extracted at 30-35 °C with dichloromethane (82 ml) and then again with dichloromethane (56 ml). Organic layers were collected and washed at 30-35 °C, with a solution of 20% aq. NaCl (2x6.5 ml). Volatiles were distilled until internal temperature 75 °C, then 40 to 80 ml of solvent was distilled under vacuum. Water (280 ml) was added. 36% HCl was added until pH 3.5±0.2 (15.38 g). The aqueous layer was separated, charcoal (1.69 g) was added, the mixture was filtered and the filter was washed with water (18 ml).

The aqueous layer was extracted with dichloromethane (2 X 34 ml) at 30-35 °C. 30% NaOH (23.0 g) was added to the aqueous layer, maintaining the temperature below 35 °C. dichloromethane was added (101 ml) and the organic layer is separated at 30-35 °C and concentrated under vacuum until 70 °C was reached, to give 37.9 g of Levomilnacipran base as yellow crude oil.

HPLC purity: 95.56%.

### Example 2

### (Process of the invention for synthesizing levomilnacipran base via 1) triethylamine and methanesulfonyl chloride in the presence of toluene)

(1S,2R)-N,N-diethyl-2-(hydroxymethyl)-1-phenylcyclopropanecarboxamide (24.7 g) was charged into a 4 neck round bottom flask at RT, under nitrogen, followed by toluene (99 ml), triethylamine (11.17 g). The mixture was cooled to 0-5 °C, methanesulfonyl chloride (12.07 g) was added over 0.5 hour, maintaining the temperature at 0-10 °C. A slurry of potassium phthalimide (39.63 g) and toluene (99 ml) was charged over a period of 30 min, then the mixture was heated to 70 °C and hold at that temperature for 12 hours. The mixture was cooled to 15-20 °C, aq. 70% EtNH2 (45.18 g) was added maintaining the same temperature. The mixture was hold at 15-20 °C for 2 hours, water (120 ml) was added and the mixture was heated to 40-42 °C for 24 hours. 30% NaOH (28.52 g) was added, the organic layer was separated and the aqueous layer was extracted with dichloromethane (41 ml) then again with dichloromethane (28 ml), then again with dichloromethane (15 ml). Organic layers were collected and washed at 30-35 °C, with a solution of 20% aq. NaCl (2x3.3 ml). Volatiles were distilled under vacuum, toluene (100 ml) and water (140 ml) were added. 36% HCl was added until pH 3.5±0.2 (8.20 g). The aqueous layer was separated at 40-42 °C, charcoal (0.85 g) was added, the mixture was filtered and the filter was washed with water (10 ml).

The aqueous layer was extracted with dichloromethane (2 X 17 ml) at 30-35 °C. 30% NaOH (12.28 g) was added maintaining the temperature below 35 °C. dichloromethane was added (50 ml) and the organic layer was separated at 30-35 °C and concentrated under vacuum until 70 °C was reached to give 19.8 g of crude oil.

HPLC purity: 94.80%.

### Example 3

### Preparation levomilnacipran hydrochloride (first solvent: methyl isobutyl ketone (MIBK) - second solvent: acetone - aqueous HCl)

A) Levomilnacipran base obtained as crude oil (8.5g) was charged into a 4 neck round bottom flask followed by MIBK (84 ml) at 20-25 °C. 37% HCl was added until pH 2.5-3, then additional 37% HCl (6% of the amount used to get to pH 2.5-3) was added. Solvent was distilled until collecting 20 ml. The mixture was cooled to 20-25 °C, acetone (18 ml) was added, the mixture was stirred for at least 1 hour, filtered, washed with acetone (18 ml), dried under vacuum to give 8.23 g of title compound as white solid.

HPLC purity: >99.99%.

Chiral purity: <0.01 % of dextro enantiomer.

### B) Preparation levomilnacipran hydrochloride (first solvent: methyl isobutyl ketone (MIBK) - second solvent: acetone - aqueous HCl)

Levomilnacipran base obtained as crude oil (9.5 g) was charged into a 4 neck round bottom flask followed by MIBK (94 ml) at 20-25 °C; 37% HCl was added until pH 2.5-3, then additional 37% HCl (6% of the amount used to get to pH 2.5-3) was added. The mixture was heated to 80-85 °C, water was added until a clear solution was obtained. The mixture was cooled to 30-40 °C while precipitation occurred. Solvent was distilled under vacuum until collecting 36 ml, the mixture was cooled to 20-25 °C, acetone (20 ml) was added, the mixture was stirred for at least 1 hour, filtered, washed with acetone (20 ml), dried under vacuum to give 9.6 g of title compound as white solid.

HPLC purity: 99.79%.

Chiral purity: <0.01 % of dextro enantiomer.

### C) Preparation levomilnacipran hydrochloride (first solvent: methyl isobutyl ketone (MIBK) - second solvent: acetone - aqueous HCl)

Levomilnacipran base obtained as crude oil (9.5 g) was charged into a 4 neck round bottom flask followed by MIBK (94 ml) at 20-25 °C; 37% HCl was added until pH 2.5-3, then additional 37% HCl (6% of the amount used to get to pH 2.5-3) was added. Solvent was distilled under vacuum until collecting 36 ml, the mixture was cooled to 20-25 °C, acetone (20 ml) was added, the mixture was stirred for at least 1 hour, filtered, washed with acetone (20 ml), dried under vacuum to give 8.6 g of title compound as white solid.

HPLC purity: 99.98%.

Chiral purity: <0.01 % of dextro enantiomer.

### D) Preparation levomilnacipran hydrochloride (first solvent methyl isobutyl ketone (MIBK) - second solvent: acetone - gaseous HCl)

Levomilnacipran base obtained as crude oil (8.5 g) was charged into a 4 neck round bottom flask followed by MIBK (84 ml), at 20-25 °C. Gaseous HCl was added until pH 1-2. The mixture was stirred at 20-25 °C for at least 1 hour, filtered, washed with acetone (18 ml), dried under vacuum to give 7.85 g of title compound as white solid.

HPLC purity: 99.98%.

Chiral purity: <0.01 % of dextro enantiomer.

### E) Preparation levomilnacipran hydrochloride (first solvent isopropanol - second solvent: methyl isobutyl ketone (MIBK) - gaseous HCl)

Levomilnacipran base obtained as crude oil (8.5 g) was charged into a 4 neck round bottom flask followed by isopropanol (45 ml) at room temperature. The mixture was heated to 50-60 °C, then gaseous HCl is added until pH 1-2. Solvent was distilled until collecting 23 ml. MIBK (111 ml) was added, the mixture was cooled to 20-25 °C, hold at that temperature for at least 1 hour, filtered, washed with MIBK (18 ml) and dried under vacuum to give 8.10 g of title compound as white solid.

HPLC purity: 99.97%.

Chiral purity: <0.01 % of of dextro enantiomer.

### F) Preparation levomilnacipran hydrochloride (first solvent isobutanol - second solvent: ethyl acetate - gaseous HCl)

Levomilnacipran base obtained as crude oil (8.57 g) was charged into a 4 neck round bottom flask followed by isobutanol (84 ml) at room temperature. The mixture was heated to 60-70 °C, then gaseous HCl was added until pH 1-2. Solvent was distilled until collecting 60 ml. Ethyl acetate (123 ml) was added, the mixture was cooled to 20-25 °C, hold at that temperature for at least 1 hour, filtered, washed with AcOEt (18 ml) and dried under vacuum to give 7.37 g of title compound as white solid.

HPLC purity: 99.97%.

Chiral purity: <0.01 % of of dextro enantiomer.

### G) Preparation levomilnacipran hydrochloride (first solvent isobutanol - second solvent: ethyl acetate - aqueous HCl)

Levomilnacipran base obtained as crude oil (9.5 g) was charged into a 4 neck round bottom flask followed by isobutanol (94 ml) at room temperature. 37% HCl was added until pH 2.5-3, then additional 37% HCl (6% of the amount used to get to pH 2.5-3) was added. Solvent was distilled until collecting 36 ml. Ethyl acetate (200 ml) was added, the mixture was cooled to 20-25 °C, hold at that temperature for at least 1 hour, filtered, washed with AcOEt (20 ml) and dried under vacuum to give 7.37 g of title compound as white solid.

HPLC purity: 99.99%.

Chiral purity: <0.01 % of of dextro enantiomer.

### H) Preparation levomilnacipran hydrochloride (first solvent isobutanol - second solvent: isobutyl acetate - aqueous HCl)

Levomilnacipran base obtained as crude oil (8.5 g) was charged into a 4 neck round bottom flask followed by isobutanol (84.1 ml) at room temperature. 37% HCl was added until pH 2.5-3, then additional 37% HCl (6% of the amount used to get to pH 2.5-3) was added. Solvent was distilled until collecting 60 ml. Isobutylacetate (50 ml) was added at 100±10 °C, the mixture was cooled to 20-25 °C, additional isobutylacetate (50 ml) was added, the mixture was hold at 20-25 °C for at least 1 hour, filtered, washed with isobutylacetate (18 ml) and dried under vacuum to give 7.78 g of title compound as white solid.

HPLC purity: 99.84%.

Chiral purity: 0.05% of of dextro enantiomer.

### Example 4

### Preparation of levomilna-alcohol of formula II (1S,2R)-N,N-diethyl-2-(hydroxymethyl)-1-phenylcyclopropanecarboxamide

Sodium amide (130.8 g) was charged into a 4 neck round bottom flask, under nitrogen atmosphere, followed by toluene (1.00 L). The mixture was cooled to 0-5 °C and a solution of phenylacetonitrile (400.0 g) in toluene (46 ml) was added, while maintaining the temperature between 0 °C and 15 °C. After the addition the mixture was hold at 10 ± 2 °C for 1 hour. A solution of R-(-)-epichlorhydrin (106.4 ml) in toluene (92 ml) was added while maintaining the temperature at 10 ± 5 °C. The reaction mixture was then hold at 10 ± 2 °C for at least 2 hours. Then it was poured on cold water (560 ml), maintaining the temperature below 40 °C. After distillation under vacuum of about 1.20 L, the mixture was heated to 95±5 °C and 30% NaOH (536.0 g) was added over a period of at least 1.5 h. The mixture was hold at 95±5 °C for additional 4 h, then cooled to 55±5 °C. Water (300 ml) and toluene (440 ml) were added, the aqueous layer was separated and further washed with toluene (440 ml). To the obtained aqueous layer toluene (640 ml) was added and the mixture was cooled to 25±5 °C. 37% HCl was added maintaining the temperature at 25±5 °C until pH 1-2. The mixture was heated to 60±5 °C and hold at that temperature for 3 hours; it was then cooled to 20-25 °C, the aqueous phase was separated, water (400 ml) was added and aq. Na₂CO₃ was added until pH 8-9. The aqueous layer was separated and the organic layer was washed with water (400 ml). The organic layer was concentrated under vacuum until reaching 70-75 °C. Isopropanol (292 ml) was added and the solution was cooled to 30±5 °C. If spontaneous precipitation did not occur, the mixture was seeded with (1S,5R)-1-phenyl-3-oxabicyclo[3.1.0]hexan-2-one. After precipitation occurred, the mixture was hold at 30±5 °C for 0.5 hour, then water (144 ml) was added over a period of about 1 h, while maintaining 30±5 °C. The mixture was then cooled to 0-5 °C and hold at that temperature for at least 2 hours. The mixture was filtered and washed with a mixture iPrOH/H₂O 2/1 (80 ml) at 0-5 °C and dried under vacuum to give 150 g of (1S,5R)-1-phenyl-3-oxabicyclo[3.1.0]hexan-2-one as beige solid.

HPLC purity: 99.95%

Chiral purity: 0.14% of dextro enantiomer

Aluminium trichloride (127.91 g) was charged into a 4 neck round bottom flask, at room temperature, under nitrogen atmosphere, followed by dichloromethane (413 ml). The mixture was cooled to 0-5 °C, then a solution of (1S,5R)-1-phenyl-3-oxabicyclo[3.1.0]hexan-2-one (144.0 g) in dichloromethane (269 ml) was added while maintaining the temperature at 0-10 °C. The mixture is hold at 0-10 °C for 10 minutes, then diethylamine (139.3 g) was added, maintaining the temperature at 0-10 °C. The mixture was heated to 20-25 °C and hold at that temperature for 2.5 h, then it was poured into cold water (539 ml) maintaining the temperature below 30 °C, obtaining a two layer mixture. The reaction flask was washed with dichloromethane (18 ml) and water (36 ml) and the washes were added to the two layer mixture previously obtained. The mixture was hold at 25-30 °C for 0.5 h and filtered through celite. The celite pad was washed with dichloromethane (34 ml) and water (20 ml) The aqueous layer was separated, and further extracted with dichloromethane (13 ml). The organic layers were combined, water (72 ml) and 37% HCl (1.8 ml) were added, the mixture was stirred for 10 min, the aqueous layer was separated and the organic layer was further washed with water (170 ml). Volatiles were distilled, toluene (165 ml) was added and the solvent was distilled to give 202.9 g of title compound as brown oil.

## Claims

1. A one-pot process for preparing (1S,2R)-1-phenyl-1-(N,N-diethylaminocarbonyl)-2-aminomethylcyclopropane of formula (I) 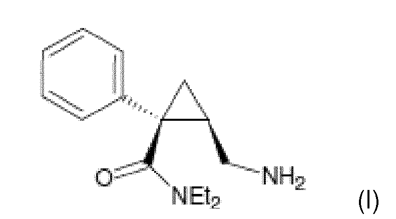 comprising the step of reacting (1S,2R)-N,N-diethyl-2-(hydroxymethyl)-1-phenylcyclopropanecarboxamide successively with the following reactants 1) triethyl orthoformate and methanesulfonic acid or triethylamine and methanesulfonyl chloride, 2) a phthalimidating agent, 3) aqueous EtNH₂, wherein the reaction is carried out in toluene.

2. The one-pot process of claim 1, wherein the reactant 1) is triethyl orthoformate and methanesulfonic acid.

3. The one-pot process of claim 1, wherein and the reactant 1) is triethylamine and methanesulfonyl chloride.

4. The one-pot process according to anyone of claims 1-3, wherein the phthalimidating agent 2) is preferably potassium phthalimide.

5. The one-pot process according to anyone of claims 1-4, wherein (1S,2R)-1-phenyl-1-(N,N-diethylaminocarbonyl)-2-aminomethylcyclopropane of formula (I) is further salified in the presence of a pharmaceutically acceptable acid, thus obtaining a pharmaceutically acceptable salt.

6. The one-pot process according to anyone of claims 1-5, wherein the pharmaceutically acceptable acid is hydrochloric acid.

7. A process for preparing (1S,2R)-1-phenyl-1-(N,N-diethylaminocarbonyl)-2-aminomethylcyclopropane hydrochloride with a combination of a first solvent and a second solvent comprising reacting (1S,2R)-1-phenyl-1-(N,N-diethylaminocarbonyl)-2-aminomethylcyclopropane with hydrochloride acid in the presence of the first solvent and the mixture so obtained is treated with the second solvent, being the combination selected from the group consisting of:
a) first solvent: methyl isobutyl ketone (MIBK) and second solvent: acetone,
b) first solvent: isopropanol and second solvent: methyl isobutyl ketone (MIBK)
c) first solvent: isobutanol and second solvent: ethyl acetate, and
d) first solvent: isobutanol and second solvent: isobutyl acetate.

8. The process according to claim 7, wherein the hydrochloric acid is either aqueous HCl or gaseous HCl.

9. A process for preparing (1S,2R)-N,N-diethyl-2-(hydroxymethyl)-1-phenylcyclopropanecarboxamide of Formula (II) 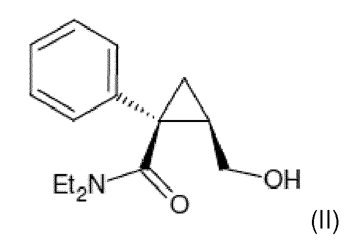 comprising the following steps:
i) reacting phenylacetonitrile with R(-)epichlorhydrin in the presence of a base containing an alkaline metal, followed by treatment of an alkaline metal hydroxide and then by a treatment with an acid, thus obtaining (1S,5R)-1-phenyl-3-oxabicyclo[3.1.0]hexan-2-one of formula III 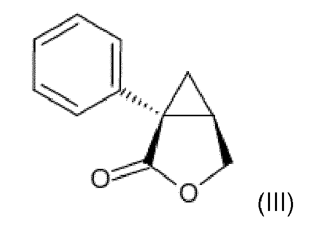
ii) crystallizing (1S,5R)-1-phenyl-3-oxabicyclo[3.1.0]hexan-2-one as crude oil in a mixture of isopropyl alcohol and water;
iii) reacting the crystallized (1S,5R)-1-phenyl-3-oxabicyclo[3.1.0]hexan-2-one with diethylamine in the presence of Lewis acid-amine complex in dichloromethane.

10. The process according to claim 9, wherein in step i) the base containing alkaline metal is NaNH₂, which is preferably reacted in the presence of toluene.

11. The process according to claim 9 or claim 10, wherein the alkaline metal hydroxide is NaOH.

12. The process according to anyone of claims 9-10, wherein in step iii) Lewis acid-amine complex is AlCl₃-NHEt₂.
